# EUROPEAN PATENT APPLICATION

(11) **EP 4 834 944 A1**
(43) Date of publication of application: **04.11.2026**
(21) Application number: 26168314.8
(22) Date of filing: 27.03.2026
(51) Int. Cl.: C07K 16/32

(54) **ANTI-HER2 ANTIBODY, METHOD FOR PRODUCING ANTI-HER2 ANTIBODY, AND METHOD FOR SUPPRESSING NON-SPECIFIC ADSORPTION OF ANTI-HER2 ANTIBODY**

(30) Priority: 28.03.2025 JP 2025056347
(71) Applicant: SYSMEX CORPORATION, Kobe-shi Hyogo 651-0073 (JP)
(72) Inventor: EGASHIRA, Yuriko, Kobe-shi, Hyogo 651-0073 (JP); FUKUNAGA, Atsushi, Kobe-shi, Hyogo 651-0073 (JP); ATARASHI, Yusuke, Kobe-shi, Hyogo 651-0073 (JP)
(74) Representative: De Clercq & Partners

(57) **Abstract**

Disclosed is an anti-HER2 antibody, in which three amino acid residues selected from the group consisting of positions 63, 65, 67, and 72 of a light chain defined by the Chothia method are changed to arginine residues, an amino acid sequence of the light chain excluding said three amino acid residues is not modified from an original amino acid sequence, and an amino acid sequence of a heavy chain is not modified.

## Description

### TECHNICAL FIELD

The present invention relates to an anti-Human Epidermal Growth Factor Receptor 2 (HER2) antibody. The present invention relates to a method for producing an anti-HER2 antibody. The present invention relates to a method for suppressing non-specific adsorption of an anti-HER2 antibody.

### BACKGROUND

HER2 is one of the receptors present on the cell membrane and is involved in the regulation of cell proliferation and differentiation. On the other hand, it has been confirmed that HER2 is overexpressed in various cancer cells. In addition, HER2 is known to be associated with an increase in malignancy, recurrence rate, and mortality rate of cancer. Trastuzumab, approved as an antineoplastic agent, is an antibody medicine that targets HER2 as a molecular target. When Trastuzumab binds to HER2 on cancer cells, the intracellular signal transduction system by HER2 is inhibited, and suppression of proliferation and apoptosis of cancer cells are induced. Furthermore, antibody-dependent cellular cytotoxicity (ADCC) of immune cells is induced by the binding of Trastuzumab to cancer cells. Trastuzumab shows a high effect particularly on breast cancer with HER2 overexpression. In recent years, the indication of an antibody-drug conjugate containing Trastuzumab and an anticancer agent has also been recognized for breast cancer with low HER2 expression. Therefore, there is an increasing need for a test capable of detecting cancer cells with a low expression level of HER2. An anti-HER2 antibody is also used for the evaluation of the expression level of HER2 in cancer cells.

### SUMMARY

In order to accurately detect cancer cells with a low expression level of HER2, non-specific adsorption of an anti-HER2 antibody to a cell that hardly expresses HER2 (hereinafter, also referred to as a "HER2-negative cell") cannot be ignored. For example, a tissue specimen collected from a cancer patient may contain not only cancer cells expressing HER2 but also HER2-negative cells such as normal cells. Therefore, when many anti-HER2 antibodies non-specifically adsorb to HER2-negative cells, the background becomes high in the detection of cancer cells expressing HER2. As a result, a problem arises that the detection sensitivity of cancer cells with a low expression level of HER2 decreases.

US Patent Application Publication No. 2018/0179298 discloses that the affinity of an antibody for an antigen is improved by modifying amino acid residues in framework region 3 (FR3) of a light chain of the antibody. US Patent Application Publication No. 2022/0204649 discloses that the affinity of an anti-HER2 antibody for an antigen is improved by modifying amino acid residues in FR3 of a heavy chain of the antibody. However, these documents do not describe suppressing non-specific adsorption of an antibody to a cell that does not express an antigen.

An object of the present invention is to provide an antibody in which non-specific adsorption to a HER2-negative cell is suppressed, i.e. lower than compared to that of a reference antibody by modifying an anti-HER2 antibody. Another object of the present invention is to provide a method for producing the anti-HER2 antibody and a method for suppressing non-specific adsorption of an anti-HER2 antibody.

The present invention relates to an isolated anti-HER2 antibody, in which three amino acid residues selected from the group consisting of positions 63, 65, 67, and 72 of a light chain defined by the Chothia method are arginine residues. In particular embodiments, said antibody has been obtained from an original anti-HER2 antibody sequence in which the three amino acid residues selected from the group consisting of positions 63, 65, 67, and 72 of a light chain defined by the Chothia method are not arginine, and changing these amino acid residues in said antibody sequence such that the three amino acid residues selected from the group consisting of positions 63, 65, 67, and 72 of a light chain defined by the Chothia method are arginine and producing said antibody. In particular embodiments, the amino acid sequence of the light chain excluding said three amino acid residues is not modified from the original amino acid sequence, and an amino acid sequence of a heavy chain is not modified. The present invention thus also relates to an anti-HER2 antibody, in which three amino acid residues selected from the group consisting of positions 63, 65, 67, and 72 of a light chain defined by the Chothia method are changed to arginine residues, an amino acid sequence of the light chain excluding said three amino acid residues is not modified from an original amino acid sequence, and an amino acid sequence of a heavy chain is not modified.

In certain embodiments, non-specific adsorption to a HER2-negative cell of the anti-HER2 antibody is suppressed, and preferably lower than compared to a reference antibody, and the reference antibody comprises a light chain represented by an amino acid sequence of SEQ ID No. 20, 21, 22, or 23 and a heavy chain represented by an amino acid sequence of SEQ ID No. 12 or 13, and the amino acid sequences of the light chain and the heavy chain are defined by the Chothia method. In certain embodiments, said three amino acid residues are changed from neutral amino acid residues to arginine residues. In certain embodiments, the neutral amino acid residues are threonine residues and/or serine residues. In certain embodiments, the light chain comprises LCDR1 represented by an amino acid sequence of SEQ ID No. 14, LCDR2 represented by an amino acid sequence of SEQ ID No. 15, and LCDR3 represented by an amino acid sequence of SEQ ID No. 16, and the heavy chain comprises HCDR1 represented by an amino acid sequence of SEQ ID No. 17, HCDR2 represented by an amino acid sequence of SEQ ID No. 18, and HCDR3 represented by an amino acid sequence of SEQ ID No. 19. In certain embodiments, the anti-HER2 antibody comprises a light chain variable region represented by an amino acid sequence of SEQ ID No. 1, 3, 5, or 7 and a heavy chain variable region represented by an amino acid sequence of SEQ ID No. 11. In certain embodiments, the original amino acid sequence of a variable region of the light chain is an amino acid sequence of SEQ ID No. 9, and an amino acid sequence of a variable region of the heavy chain is an amino acid sequence of SEQ ID No. 11. In certain embodiments, the original amino acid sequence of the light chain is an amino acid sequence of SEQ ID No. 10, and an amino acid sequence of the heavy chain is an amino acid sequence of SEQ ID No. 12 or 13. In certain embodiments, an amino acid sequence of the light chain is an amino acid sequence of SEQ ID No. 2, 4, 6, or 8, and an amino acid sequence of the heavy chain is an amino acid sequence of SEQ ID No. 12 or 13.

The present invention also relates to a method for producing an anti-HER2 antibody, comprising: generating an anti-HER2 antibody comprising an amino acid sequence of the anti-HER2 antibody using a polynucleotide encoding the amino acid sequence of the anti-HER2 antibody, and recovering the anti-HER2 antibody, wherein in the polynucleotide, codons encoding three amino acid residues selected from the group consisting of positions 63, 65, 67, and 72 of a light chain defined by the Chothia method are codons encoding arginine residues. In certain embodiments, the anti-HER2 antibody comprises a light chain variable region represented by an amino acid sequence of SEQ ID No. 1, 3, 5, or 7 and a heavy chain variable region represented by an amino acid sequence of SEQ ID No. 11. In certain embodiments, an amino acid sequence of the light chain is an amino acid sequence of SEQ ID No. 2, 4, 6, or 8, and an amino acid sequence of the heavy chain is an amino acid sequence of SEQ ID No. 12 or 13.

The present invention relates to methods for suppressing non-specific adsorption of an anti-HER2 antibody, or methods of generating an antibody in which the non-specific adsorption has been suppressed, which methods comprise obtaining an anti-HER2 antibody or antibody sequence, wherein in its original sequence, three amino acid residues selected from the group consisting of positions 63, 65, 67, and 72 of a light chain defined by the Chothia method are not arginine; and suppressing non-specific adsorption to a HER2-negative cell of said antibody by changing three amino acid residues in framework region 3 selected from the group consisting of positions 63, 65, 67, and 72 of a light chain defined by the Chothia method to arginine residues in the anti-HER2 antibody and producing said antibody. The present invention thus also relates to a method for suppressing non-specific adsorption of an anti-HER2 antibody, comprising: suppressing non-specific adsorption to a HER2-negative cell, and preferably making it lower than that of a reference antibody by changing three amino acid residues in framework region 3 defined by the Chothia method to arginine residues in the anti-HER2 antibody, wherein said three amino acid residues are selected from the group consisting of positions 63, 65, 67, and 72 of a light chain defined by the Chothia method, and the reference antibody comprises a light chain represented by an amino acid sequence of SEQ ID No. 20, 21, 22, or 23 and a heavy chain represented by an amino acid sequence of SEQ ID No. 12 or 13, and the amino acid sequences of the light chain and the heavy chain are defined by the Chothia method.

In certain embodiments, the original amino acid sequence of a variable region of the light chain of said anti-HER antibody is an amino acid sequence of SEQ ID No. 9, and an amino acid sequence of a variable region of the heavy chain is an amino acid sequence of SEQ ID No. 11. the original amino acid sequence of the light chain is an amino acid sequence of SEQ ID No. 10, and an amino acid sequence of the heavy chain is an amino acid sequence of SEQ ID No. 12 or 13. In certain embodiments, the anti-HER2 antibody obtained by said method comprises a light chain variable region represented by an amino acid sequence of SEQ ID No. 1, 3, 5, or 7 and a heavy chain variable region represented by an amino acid sequence of SEQ ID No. 11. In certain embodiments, an amino acid sequence of the light chain of the antibody obtained by said method is an amino acid sequence of SEQ ID No. 2, 4, 6, or 8, and an amino acid sequence of the heavy chain is an amino acid sequence of SEQ ID No. 12 or 13.

According to the present invention, by modifying an anti-HER2 antibody, an antibody in which non-specific adsorption to a HER2-negative cell is suppressed, and lower when compared to a reference antibody, a method for producing the anti-HER2 antibody, and a method for suppressing non-specific adsorption of an anti-HER2 antibody are provided.

### BRIEF DESCRIPTION OF THE DRAWINGS

Fig. 1 is a histogram showing the results of analyzing non-specific adsorption of an anti-HER2 antibody to a HER2-negative cell by flow cytometry.
Fig. 2 is a graph showing the average value of fluorescence intensity in the histogram of Fig. 1.
Fig. 3 is a chromatogram showing the results of analyzing non-specific adsorption of an anti-HER2 antibody to a heparin column by chromatography.

### DESCRIPTION OF THE EMBODIMENTS

In the present specification, "antibody" includes not only the form of immunoglobulin but also antibody fragments. The antibody in the form of immunoglobulin is preferably IgG. Examples of the antibody fragment include Fab, F(ab')₂, Fab', Fv, single chain antibody (scFv), reduced IgG (rIgG), diabody, and triabody.

The anti-HER2 antibody of the present invention is an antibody (hereinafter, also referred to as a "variant") in which predetermined amino acid residues in a light chain of an original anti-HER2 antibody are modified to arginine. More specifically, in the anti-HER2 antibody of the present invention, three amino acid residues selected from the group consisting of positions 63, 65, 67, and 72 of a light chain defined by the Chothia method (hereinafter, also referred to as "predetermined amino acid residues of a light chain") are arginine residues. The original anti-HER2 antibody (hereinafter, also referred to as an "unmodified antibody") is an anti-HER2 antibody before the predetermined amino acid residues of the light chain are modified. In the unmodified antibody, the predetermined amino acid residues of the light chain are amino acid residues other than arginine residues.

Positions 63, 65, 67, and 72 of a light chain defined by the Chothia method are amino acid residues in FR3 of the light chain. FR is a region other than a complementarity determining region (CDR) present in each variable region of a light chain and a heavy chain of an antibody. FR plays a role of a scaffold connecting three CDRs and contributes to the structural stability of the CDRs. Therefore, the amino acid sequence of FR is highly conserved among antibodies of the same species. In each variable region of a light chain and a heavy chain, there are three CDRs, CDR1, CDR2, and CDR3, and four FRs, FR1, FR2, FR3, and FR4. These are arranged in the order of FR1, CDR1, FR2, CDR2, FR3, CDR3, and FR4 from the N-terminal side of the variable region.

The Chothia method is a kind of method for numbering amino acid residues of CDR for defining the boundary and length of CDR (Chothia C. and Lesk AM., Canonical Structures for the Hypervariable Regions of Immunoglobulins., J Mol Biol., vol.196, pp.901-917, 1987). When amino acid residues of CDR are numbered by the Chothia method, amino acid residues of FR are also numbered.

In the anti-HER2 antibody of the present invention, the combination of predetermined amino acid residues of the light chain is as follows:
a) amino acid residues at positions 63, 65, and 67 of a light chain defined by the Chothia method;
b) amino acid residues at positions 63, 65, and 72 of a light chain defined by the Chothia method;
c) amino acid residues at positions 63, 67, and 72 of a light chain defined by the Chothia method; and
d) amino acid residues at positions 65, 67, and 72 of a light chain defined by the Chothia method.

The anti-HER2 antibody of the present invention in which the three amino acid residues of a) above are arginine residues (hereinafter, also referred to as "63,65,67 variant") comprises, for example, a light chain variable region represented by the amino acid sequence of SEQ ID No. 1. In addition, the amino acid sequence of the light chain of the 63,65,67 variant may be, for example, the amino acid sequence of SEQ ID No. 2. These amino acid sequences are shown below. The underlines indicate positions 63, 65, and 67 of the light chain defined by the Chothia method.
[Light chain variable region of 63,65,67 variant] 
DIQMTQSPSSLSASVGDRVTITCRASQDVNTAVAWYQQKPGKAPKLLIYSASFLYSGVPSRFR
GRRRGTDFTLTISSLQPEDFATYYCQQHYTTPPTFGQGTKVEIKRTV (SEQ ID No. 1)
[Light chain of 63,65,67 variant]

DIQMTQSPSSLSASVGDRVTITCRASQDVNTAVAWYQQKPGKAPKLLIYSASFLYSGVPSRFR
GRRRGTDFTLTISSLQPEDFATYYCQQHYTTPPTFGQGTKVEIKRTVAAPSVFIFPPSDEQLKS
GTASVVCLLNNFYPREAKVQWKVDNALQSGNSQESVTEQDSKDSTYSLSSTLTLSKADYEK
HKVYACEVTHQGLSSPVTKSFNRGEC (SEQ ID No. 2)

The anti-HER2 antibody of the present invention in which the three amino acid residues of b) above are arginine residues (hereinafter, also referred to as "63,65,72 variant") comprises, for example, a light chain variable region represented by the amino acid sequence of SEQ ID No. 3. In addition, the amino acid sequence of the light chain of the 63,65,72 variant may be, for example, the amino acid sequence of SEQ ID No. 4. These amino acid sequences are shown below. The underlines indicate positions 63, 65, and 72 of the light chain defined by the Chothia method.
[Light chain variable region of 63,65,72 variant] 
DIQMTQSPSSLSASVGDRVTITCRASQDVNTAVAWYQQKPGKAPKLLIYSASFLYSGVPSRFR
GRRSGTDFRLTISSLQPEDFATYYCQQHYTTPPTFGQGTKVEIKRTV (SEQ ID No. 3)
[Light chain of 63,65,72 variant]

DIQMTQSPSSLSASVGDRVTITCRASQDVNTAVAWYQQKPGKAPKLLIYSASFLYSGVPSRFR
GRRSGTDFRLTISSLQPEDFATYYCQQHYTTPPTFGQGTKVEIKRTVAAPSVFIFPPSDEQLKS
GTASVVCLLNNFYPREAKVQWKVDNALQSGNSQESVTEQDSKDSTYSLSSTLTLSKADYEK
HKVYACEVTHQGLSSPVTKSFNRGEC (SEQ ID No. 4)

The anti-HER2 antibody of the present invention in which the three amino acid residues of c) above are arginine residues (hereinafter, also referred to as "63,67,72 variant") comprises, for example, a light chain variable region represented by the amino acid sequence of SEQ ID No. 5. In addition, the amino acid sequence of the light chain of the 63,67,72 variant may be, for example, the amino acid sequence of SEQ ID No. 6. These amino acid sequences are shown below. The underlines indicate positions 63, 67, and 72 of the light chain defined by the Chothia method.
[Light chain variable region of 63,67,72 variant] 
DIQMTQSPSSLSASVGDRVTITCRASQDVNTAVAWYQQKPGKAPKLLIYSASFLYSGVPSRFR
GSRRGTDFRLTISSLQPEDFATYYCQQHYTTPPTFGQGTKVEIKRTV (SEQ ID No. 5)
[Light chain of 63,67,72 variant]

DIQMTQSPSSLSASVGDRVTITCRASQDVNTAVAWYQQKPGKAPKLLIYSASFLYSGVPSRFR
GSRRGTDFRLTISSLQPEDFATYYCQQHYTTPPTFGQGTKVEIKRTVAAPSVFIFPPSDEQLKS
GTASVVCLLNNFYPREAKVQWKVDNALQSGNSQESVTEQDSKDSTYSLSSTLTLSKADYEK
HKVYACEVTHQGLSSPVTKSFNRGEC (SEQ ID No. 6)

The anti-HER2 antibody of the present invention in which the three amino acid residues of d) above are arginine residues (hereinafter, also referred to as "65,67,72 variant") comprises, for example, a light chain variable region represented by the amino acid sequence of SEQ ID No. 7. In addition, the amino acid sequence of the light chain of the 65,67,72 variant may be, for example, the amino acid sequence of SEQ ID No. 8. These amino acid sequences are shown below. The underlines indicate positions 65, 67, and 72 of the light chain defined by the Chothia method.
[Light chain variable region of 65,67,72 variant] DIQMTQSPSSLSASVGDRVTITCRASQDVNTAVAWYQQKPGKAPKLLIYSASFLYSGVPSRFS GRRRGTDFRLTISSLQPEDFATYYCQQHYTTPPTFGQGTKVEIKRTV (SEQ ID No. 7)
[Light chain of 65,67,72 variant]

DIQMTQSPSSLSASVGDRVTITCRASQDVNTAVAWYQQKPGKAPKLLIYSASFLYSGVPSRFS
GRRRGTDFRLTISSLQPEDFATYYCQQHYTTPPTFGQGTKVEIKRTVAAPSVFIFPPSDEQLKS
GTASVVCLLNNFYPREAKVQWKVDNALQSGNSQESVTEQDSKDSTYSLSSTLTLSKADYEK
HKVYACEVTHQGLSSPVTKSFNRGEC (SEQ ID No. 8)

It is preferred that the nucleotide sequence of the gene encoding the variable regions of the light chain and heavy chain of the unmodified antibody is known. Alternatively, it is preferred that a hybridoma producing the antibody is available for the unmodified antibody. The nucleotide sequence of the gene encoding the unmodified antibody can be determined from the gene of the hybridoma. The unmodified antibody is preferably a monoclonal antibody. The unmodified antibody may be an antibody derived from any animal. Examples thereof include antibodies derived from mice, rats, hamsters, rabbits, goats, horses, chickens, humans, and the like. The unmodified antibody may be a chimeric antibody or a humanized antibody. A chimeric antibody is an antibody derived from animal species in which the variable regions of the light chain and heavy chain and the constant region are different from each other. A humanized antibody is an antibody in which the CDRs of the light chain and heavy chain are derived from an animal species other than human, and the remaining regions other than the CDRs are derived from human. A particularly preferred unmodified antibody is Trastuzumab.

In the unmodified antibody, the predetermined amino acid residues of the light chain are preferably neutral amino acid residues. Neutral amino acid residues are alanine residues, asparagine residues, isoleucine residues, glycine residues, glutamine residues, cysteine residues, threonine residues, serine residues, tyrosine residues, phenylalanine residues, proline residues, valine residues, methionine residues, leucine residues, and tryptophan residues. Preferably, the predetermined amino acid residues of the light chain in the unmodified antibody are threonine residues and/or serine residues.

In the anti-HER2 antibody of the present invention, the amino acid sequence of the light chain excluding the predetermined amino acid residues of the light chain is not modified from the original amino acid sequence (in other words, the amino acid sequence of the light chain excluding the predetermined amino acid residues of the light chain is identical to the original amino acid sequence). The "original amino acid sequence" is the amino acid sequence of the unmodified antibody. That is, the amino acid sequence of the light chain of the anti-HER2 antibody of the present invention is the same as the amino acid sequence of the light chain of the unmodified antibody, except that the predetermined amino acid residues of the light chain are arginine residues. The unmodified antibody comprises, for example, a light chain variable region represented by the amino acid sequence of SEQ ID No. 9. In this case, the original amino acid sequence of the light chain variable region of the anti-HER2 antibody of the present invention is the amino acid sequence of SEQ ID No. 9. In addition, the amino acid sequence of the light chain of the unmodified antibody may be, for example, the amino acid sequence of SEQ ID No. 10. In this case, the original amino acid sequence of the light chain of the anti-HER2 antibody of the present invention is the amino acid sequence of SEQ ID No. 10. The amino acid sequences of SEQ ID Nos. 9 and 10 are the amino acid sequences of Trastuzumab. These amino acid sequences are shown below. The underlines indicate positions 63, 65, 67, and 72 of the light chain defined by the Chothia method.
[Light chain variable region of unmodified antibody] 
DIQMTQSPSSLSASVGDRVTITCRASQDVNTAVAWYQQKPGKAPKLLIYSASFLYSGVPSRFS
GSRSGTDFTLTISSLQPEDFATYYCQQHYTTPPTFGQGTKVEIKRTV (SEQ ID No. 9)
[Light chain of unmodified antibody]

DIQMTQSPSSLSASVGDRVTITCRASQDVNTAVAWYQQKPGKAPKLLIYSASFLYSGVPSRFS
GSRSGTDFTLTISSLQPEDFATYYCQQHYTTPPTFGQGTKVEIKRTVAAPSVFIFPPSDEQLKSG
TASVVCLLNNFYPREAKVQWKVDNALQSGNSQESVTEQDSKDSTYSLSSTLTLSKADYEKH
KVYACEVTHQGLSSPVTKSFNRGEC (SEQ ID No. 10)

In the anti-HER2 antibody of the present invention, the amino acid sequence of the heavy chain is not modified. That is, the amino acid sequence of the heavy chain of the anti-HER2 antibody of the present invention is the same as the amino acid sequence of the heavy chain of the unmodified antibody. The anti-HER2 antibody of the present invention and the unmodified antibody comprise, for example, a heavy chain variable region represented by the amino acid sequence of SEQ ID No. 11. When the anti-HER2 antibody of the present invention and the unmodified antibody are IgG, the amino acid sequence of the heavy chain may be, for example, the amino acid sequence of SEQ ID No. 12. In addition, when the anti-HER2 antibody of the present invention and the unmodified antibody are Fab, the amino acid sequence of the heavy chain may be, for example, the amino acid sequence of SEQ ID No. 13. The amino acid sequences of SEQ ID Nos. 11 to 13 are the amino acid sequences of Trastuzumab. These amino acid sequences are shown below.
[Heavy chain variable region] 


(SEQ ID No. 11)
[Heavy chain of IgG] 
EVQLVESGGGLVQPGGSLRLSCAASGFNIKDTYIHWVRQAPGKGLEWVARIYPTNGYTRYA
DSVKGRFTISADTSKNTAYLQMNSLRAEDTAVYYCSRWGGDGFYAMDYWGQGTLVTVSSA
STKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSREEMTKNQVSL
TCLVKGFYPSDIAVEWESNGQPENNYKTTPPVLDSDGSFFLYSKLTVDKSRWQQGNVFSCSV
 MHEALHNHYTQKSLSLSPGK (SEQ ID No. 12)
[Heavy chain of Fab]

EVQLVESGGGLVQPGGSLRLSCAASGFNIKDTYIHWVRQAPGKGLEWVARIYPTNGYTRYA
DSVKGRFTISADTSKNTAYLQMNSLRAEDTAVYYCSRWGGDGFYAMDYWGQGTLVTVSSA
STKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKT (SEQ ID No. 13)

The anti-HER2 antibody of the present embodiment preferably comprises a light chain variable region represented by an amino acid sequence of SEQ ID No. 1, 3, 5, or 7 and a heavy chain variable region represented by an amino acid sequence of SEQ ID No. 11.

In the anti-HER2 antibody of the present invention, the amino acid sequence of CDR is not modified. Therefore, the anti-HER2 antibody of the present invention contains the same CDRs as the unmodified antibody. The amino acid sequence of CDR is not particularly limited as long as it can form an antigen-binding site capable of specifically binding to HER2. In an antigen-binding site constituted by one light chain and one heavy chain, there are a total of six CDRs, LCDR1, LCDR2, LCDR3, HCDR1, HCDR2, and HCDR3. For example, the light chain of the anti-HER2 antibody of the present invention may comprise LCDR1 represented by an amino acid sequence of SEQ ID No. 14, LCDR2 represented by an amino acid sequence of SEQ ID No. 15, and LCDR3 represented by an amino acid sequence of SEQ ID No. 16. In addition, the heavy chain of the anti-HER2 antibody of the present invention may comprise HCDR1 represented by an amino acid sequence of SEQ ID No. 17, HCDR2 represented by an amino acid sequence of SEQ ID No. 18, and HCDR3 represented by an amino acid sequence of SEQ ID No. 19. The amino acid sequences of SEQ ID Nos. 14 to 19 are the amino acid sequences of Trastuzumab. These amino acid sequences are shown below.
- LCDR1: RASQDVNTAVA (SEQ ID No. 14)
- LCDR2: SASFLYS (SEQ ID No. 15)
- LCDR3: QQHYTTPPT (SEQ ID No. 16)
- HCDR1: GFNIKDT (SEQ ID No. 17)
- HCDR2: YPTNGY (SEQ ID No. 18)
- HCDR3: WGGDGFYAMDY (SEQ ID No. 19)

The anti-HER2 antibody of the present invention is preferably an antibody comprising a light chain represented by an amino acid sequence of SEQ ID No. 2, 4, 6, or 8 and a heavy chain represented by an amino acid sequence of SEQ ID No. 12 or 13. In the antibody, the amino acid sequences of the light chain and the heavy chain are defined by the Chothia method.

In the anti-HER2 antibody of the present invention, non-specific adsorption to a HER2-negative cell is limited, and is suppressed or lower compared to a reference antibody. As described above, the HER2-negative cell is a cell that hardly expresses HER2. Examples of the HER2-negative cell include human normal cells and cells derived from mammals other than humans. It is known that the HER2 expression level in human normal cells is extremely low compared to cancer cells. Examples of normal cells include peripheral blood mononuclear cells (PBMC), vascular endothelial cells, and epithelial cells. Non-specific adsorption refers to interactions other than binding by antigen-antibody reaction. Examples of non-specific adsorption include, but are not limited to, electrostatic interaction and hydrophobic interaction.

The reference antibody is a kind of antibody in which predetermined amino acid residues in the light chain of the unmodified antibody are (modified to) arginine, similarly to the anti-HER2 antibody of the present invention, however at different positions. More specifically, the reference antibody comprises a light chain represented by an amino acid sequence of SEQ ID No. 20, 21, 22, or 23 and a heavy chain represented by an amino acid sequence of SEQ ID No. 12 or 13, and the amino acid sequences of the light chain and the heavy chain are defined by the Chothia method. When the amino acid sequence of the heavy chain of the reference antibody is the amino acid sequence of SEQ ID No. 12, the reference antibody is IgG. When the amino acid sequence of the heavy chain of the reference antibody is the amino acid sequence of SEQ ID No. 13, the reference antibody is Fab.

A reference antibody comprising a light chain represented by the amino acid sequence of SEQ ID No. 20 and a heavy chain represented by the amino acid sequence of SEQ ID No. 12 or 13 is hereinafter also referred to as "63,65,70 variant". The amino acid sequence of SEQ ID No. 20 is shown below. The underlines indicate positions 63, 65, and 70 of the light chain defined by the Chothia method.

### [Light chain of 63,65,70 variant]

DIQMTQSPSSLSASVGDRVTITCRASQDVNTAVAWYQQKPGKAPKLLIYSASFLYSGVPSRFR
GRRSGTRFTLTISSLQPEDFATYYCQQHYTTPPTFGQGTKVEIKRTVAAPSVFIFPPSDEQLKS
GTASVVCLLNNFYPREAKVQWKVDNALQSGNSQESVTEQDSKDSTYSLSSTLTLSKADYEK
HKVYACEVTHQGLSSPVTKSFNRGEC (SEQ ID No. 20)

A reference antibody comprising a light chain represented by the amino acid sequence of SEQ ID No. 21 and a heavy chain represented by the amino acid sequence of SEQ ID No. 12 or 13 is hereinafter also referred to as "63,67,70 variant". The amino acid sequence of SEQ ID No. 21 is shown below. The underlines indicate positions 63, 67, and 70 of the light chain defined by the Chothia method.

### [Light chain of 63,67,70 variant]

DIQMTQSPSSLSASVGDRVTITCRASQDVNTAVAWYQQKPGKAPKLLIYSAFLYSGVPSRFR
GSRRGTRFTLTISSLQPEDFATYYCQQHYTTPPTFGQGTKVEIKRTVAAPSVFIFPPSDEQLKS
GTASVVCLLNNFYPREAKVQWKVDNALQSGNSQESVTEQDSKDSTYSLSSTLTLSKADYEK
HKVYACEVTHQGLSSPVTKSFNRGEC (SEQ ID No. 21)

A reference antibody comprising a light chain represented by the amino acid sequence of SEQ ID No. 22 and a heavy chain represented by the amino acid sequence of SEQ ID No. 12 or 13 is hereinafter also referred to as "65,67,70 variant". The amino acid sequence of SEQ ID No. 22 is shown below. The underlines indicate positions 65, 67, and 70 of the light chain defined by the Chothia method.

### [Light chain of 65,67,70 variant]

DIQMTQSPSSLSASVGDRVTITCRASQDVNTAVAWYQQKPGKAPKLLIYSASFLYSGVPSRFS
GRRRGTRFTLTISSLQPEDFATYYCQQHYTTPPTFGQGTKVEIKRTVAAPSVFIFPPSDEQLKS
GTASVVCLLNNFYPREAKVQWKVDNALQSGNSQESVTEQDSKDSTYSLSSTLTLSKADYEK
HKVYACEVTHQGLSSPVTKSFNRGEC (SEQ ID No. 22)

A reference antibody comprising a light chain represented by an amino acid sequence of SEQ ID No. 23 and a heavy chain represented by an amino acid sequence of SEQ ID No. 12 or 13 is hereinafter also referred to as "65,70,72 variant". The amino acid sequence of SEQ ID No. 23 is shown below. The underlines indicate positions 65, 70, and 72 of the light chain defined by the Chothia method.

### [Light chain of 65,70,72 variant]

DIQMTQSPSSLSASVGDRVTITCRASQDVNTAVAWYQQKPGKAPKLLIYSASFLYSGVPSRFS
GRRSGTRFRLTISSLQPEDFATYYCQQHYTTPPTFGQGTKVEIKRTVAAPSVFIFPPSDEQLKS
GTASVVCLLNNFYPREAKVQWKVDNALQSGNSQESVTEQDSKDSTYSLSSTLTLSKADYEK
HKVYACEVTHQGLSSPVTKSFNRGEC (SEQ ID No. 23)

Non-specific adsorption to a HER2-negative cell is lower or suppressed in the anti-HER2 antibody of the present invention compared to any of the reference antibodies of the 63,65,70 variant, the 63,67,70 variant, the 65,67,70 variant, and the 65,70,72 variant. Non-specific adsorption to a HER2-negative cell can be evaluated by a flow cytometry method in which each of the anti-HER2 antibody of the present invention and the reference antibody is detected with a fluorescently labeled secondary antibody, as shown in Example 3 described later.

Non-specific adsorption of the anti-HER2 antibody of the present invention and the reference antibody to a HER2-negative cell may be evaluated by affinity chromatography using a heparin column, as shown in Example 4 described later. A structure called "glycocalyx" containing heparan sulfate as a main constituent exists on the cell membrane of vascular endothelial cells. Since heparan sulfate is structurally similar to heparin, it is known that the binding property between an antibody and a vascular endothelial cell can be predicted using a heparin column (see Kraft T.E. et al., mAbs, vol.12, e1683432, 2020). The heparin column is a column packed with a resin to which heparin is covalently bonded, and is commercially available.

The anti-HER2 antibody of the present invention may comprise an additional oligopeptide or polypeptide, if necessary. Examples of such an oligopeptide and polypeptide include a signal peptide and a protein tag. The protein tag is preferably a tag that can be used for antibody purification. The protein tag can be selected from known protein tags such as, for example, a histidine (His) tag, a glutathione-S-transferase (GST) tag, and a FLAG (registered trademark) tag. The amino acid sequence of the His tag may be, for example, GSCGGHHHHHH (SEQ ID No. 24).

A known substance may be conjugated to the anti-HER2 antibody of the present invention, if necessary. Such a substance may be, for example, a labeling substance, a drug, or the like. Examples of the labeling substance include a fluorescent dye, a fluorescent protein, an enzyme, biotin, and a radioisotope. Examples of the drug include an anticancer agent, a nucleic acid medicine, a peptide medicine, and an antibiotic.

The anti-HER2 antibody of the present invention can be used in the same manner as the unmodified antibody. The anti-HER2 antibody of the present invention can be used for, for example, an antineoplastic agent, a test reagent, a test, research, and the like.

A further aspect provides a method for producing the anti-HER2 antibody of the present invention (hereinafter, also referred to as "production method of the present invention"). In the production method of the present invention, first, an anti-HER2 antibody comprising an amino acid sequence of the anti-HER2 antibody of the present invention is generated using a polynucleotide encoding the amino acid sequence. The polynucleotide comprises a polynucleotide encoding the light chain of the anti-HER2 antibody of the present invention and a polynucleotide encoding the heavy chain of the antibody.

The polynucleotide encoding the light chain of the anti-HER2 antibody of the present invention can be prepared using the polynucleotide encoding the light chain of the unmodified antibody by a known molecular biological technique such as a DNA recombination technique. In the polynucleotide encoding the light chain of the anti-HER2 antibody of the present invention, codons encoding the predetermined amino acid residues of the light chain are codons encoding arginine residues. For example, amplification is performed by a PCR method using the polynucleotide encoding the light chain of the unmodified antibody as a template and using primers having nucleotide sequences capable of modifying the predetermined amino acid residues of the light chain to arginine residues. Thereby, the polynucleotide encoding the light chain of the anti-HER2 antibody of the present invention can be obtained. The polynucleotide encoding the heavy chain of the anti-HER2 antibody of the present invention is the same as the polynucleotide encoding the heavy chain of the unmodified antibody.

The polynucleotides encoding the light chain and the heavy chain of the unmodified antibody can be obtained by a known artificial gene synthesis method when their nucleotide sequences are known. When there is a hybridoma producing the unmodified antibody, the polynucleotides encoding each of the light chain and the heavy chain of the unmodified antibody can also be obtained from RNA extracted from the hybridoma. For example, the polynucleotides encoding each of the light chain and the heavy chain can be synthesized from the RNA by a reverse transcription reaction and a RACE (Rapid Amplification of cDNA ends) method.

The polynucleotides encoding each of the light chain and the heavy chain of the anti-HER2 antibody of the present invention are preferably incorporated into an expression vector. The type of the expression vector is not particularly limited. Examples thereof include a plasmid vector and a viral vector. The expression vector is a vector that enables expression of a protein encoded by the polynucleotide incorporated in the vector in a suitable host cell. The polynucleotide encoding the light chain and the polynucleotide encoding the heavy chain may be incorporated into one expression vector. Alternatively, the polynucleotide encoding the light chain and the polynucleotide encoding the heavy chain may be incorporated into separate expression vectors. The type of the expression vector is not particularly limited and can be determined according to the host cell. Examples thereof include an expression vector for mammalian cells, an expression vector for insect cells, an expression vector for E. coli, and an expression vector for yeast.

In the production method of the present invention, the anti-HER2 antibody of the present invention is generated by a protein expression system using the obtained polynucleotide. The protein expression system may be an expression system using a host cell. Alternatively, the protein expression system may be a cell-free protein synthesis system. Examples of the host cell include mammalian cells, insect cells, E. coli, yeast, and the like. Examples of the cell-free protein synthesis system include a wheat germ-derived synthesis system, an E. coli-derived synthesis system, and a reconstituted cell-free protein synthesis system.

In the production method of the present invention, the anti-HER2 antibody of the present invention generated by the protein expression system is recovered. The method itself for recovering the antibody generated by the protein expression system is known. For example, when the antibody is generated in the host cell, the host cell may be lysed with a solution containing a suitable solubilizing agent to liberate the antibody in the solution. When the host cell secretes the generated antibody from the inside of the cell into a medium, the culture supernatant may be recovered. In the cell-free protein synthesis system, the synthesized antibody is contained in the reaction solution. The antibody liberated in the liquid can be recovered by a known method such as affinity chromatography. For example, when the generated antibody is IgG, it can be recovered by affinity chromatography using Protein A or G. When the generated antibody has a His tag as a peptide tag, it can be recovered by affinity chromatography using a carrier containing Ni-NTA (nitrilotriacetic acid forming a chelate with a nickel ion). When the generated antibody has a GST tag as a protein tag, it can be recovered by affinity chromatography using a carrier containing glutathione. If necessary, the recovered antibody may be purified by a known method such as gel filtration or dialysis.

A further aspect provides a method for suppressing non-specific adsorption of an anti-HER2 antibody (hereinafter, also referred to as "suppression method of the present invention"). The suppression method of the present invention comprises suppressing non-specific adsorption to a HER2-negative cell compared to the unmodified/original antibody or making it lower than compared to a reference antibody by changing three amino acid residues in framework region 3 defined by the Chothia method to arginine residues in the anti-HER2 antibody. Here, the three amino acid residues are three residues selected from the group consisting of positions 63, 65, 67, and 72 of a light chain defined by the Chothia method.

The anti-HER2 antibody before the three amino acid residues are modified to arginine residues is the same as described for the unmodified antibody above. The anti-HER2 antibody in which the three amino acid residues are modified to arginine residues is the same as described for the anti-HER2 antibody of the present invention above.

The reference antibody is an anti-HER2 antibody comprising a light chain represented by an amino acid sequence of SEQ ID No. 20, 21, 22, or 23 and a heavy chain represented by an amino acid sequence of SEQ ID No. 12 or 13, wherein the amino acid sequences of the light chain and the heavy chain are defined by the Chothia method. Details of the reference antibody are as described above.

It can be confirmed, for example, by a flow cytometry method that non-specific adsorption of the modified anti-HER2 antibody to a HER2-negative cell is suppressed and lower than compared to the reference antibody. For example, for comparison to the reference antibody each of the modified anti-HER2 antibody and the reference antibody is brought into contact with a HER2-negative cell. Details of the HER2-negative cell are as described above. After the contact is performed for a predetermined time, the HER2-negative cell is washed. Thereafter, a labeled secondary antibody is brought into contact with the HER2-negative cell. The labeled secondary antibody is an antibody that specifically binds to the anti-HER2 antibody and the reference antibody, and is an antibody to which a labeling substance is conjugated. The labeling substance is as described above. A preferred labeling substance is a fluorescent dye. After the contact is performed for a predetermined time, the HER2-negative cell is washed. Then, a signal emitted from the labeled secondary antibody is detected. When a signal measurement value in the case of using the anti-HER2 antibody is lower than a signal measurement value in the case of using the reference antibody, it is shown that non-specific adsorption of the modified anti-HER2 antibody to the HER2-negative cell is suppressed compared to the reference antibody. In consideration of non-specific adsorption of the labeled secondary antibody to the HER2-negative cell, a signal measurement value may be obtained by bringing the labeled secondary antibody into contact with a HER2-negative cell that has not been in contact with the anti-HER2 antibody and the reference antibody. This signal measurement value can be used as a background value.

Alternatively, it may be confirmed by affinity chromatography using a heparin column that non-specific adsorption of the modified anti-HER2 antibody to a HER2-negative cell is suppressed compared to the reference antibody. The modified anti-HER2 antibody and the reference antibody are each applied to separate heparin columns and eluted from the columns by a conventional method. At this time, when a retention time (RT) in the case of applying the anti-HER2 antibody to the column is shorter than an RT in the case of applying the reference antibody, it is shown that non-specific adsorption of the modified anti-HER2 antibody to the HER2-negative cell is suppressed compared to the reference antibody. The retention volume may be compared instead of the retention time.

A further aspect provides a polynucleotide encoding the anti-HER2 antibody of the present invention. The polynucleotide comprises a polynucleotide encoding a light chain and a polynucleotide encoding a heavy chain. These polynucleotides may be incorporated into a suitable vector. The type of the vector is not particularly limited. Examples thereof include a plasmid vector and a viral vector. The vector may be linear or circular. The type of the plasmid vector is not particularly limited. Examples thereof include an expression vector, a vector for producing a viral vector, a transposon vector, and a cloning vector. The transposon vector is a vector that enables a polynucleotide incorporated in the transposon vector to be integrated into the genome of a host cell by being introduced into a suitable host together with an expression vector into which a gene encoding a transposase is incorporated.

The type of the viral vector is not particularly limited. Examples thereof include a retroviral vector, a lentiviral vector, an adenoviral vector, an adeno-associated virus (AAV) vector, a vaccinia virus vector, an Epstein-Barr virus (EBV) vector, and the like. It is preferred that the viral vector is replication-defective so that the virus does not self-replicate in infected cells.

The vector may comprise a suitable control sequence, if necessary. Examples of such a control sequence include a promoter sequence, an operator sequence, an enhancer sequence, a nucleotide sequence encoding a drug resistance marker, and a multi-cloning site.

A further aspect provides a host cell comprising the polynucleotide encoding the anti-HER2 antibody of the present invention. The polynucleotide and the host cell are as described above.

A further aspect provides a pharmaceutical composition comprising the anti-HER2 antibody of the present invention (hereinafter, also referred to as "pharmaceutical composition of the present invention"). The pharmaceutical composition of the present invention preferably comprises the anti-HER2 antibody of the present invention in a pharmacologically effective amount. The pharmaceutical composition of the present invention may comprise components other than the anti-HER2 antibody of the present invention. Such a component is, for example, a pharmaceutically acceptable additive. Examples of the pharmaceutically acceptable additive include a solvent, a stabilizer, a preservative, a buffer, a soothing agent, an emulsifier, a suspending agent, a solubilizer, a pH adjuster, an isotonic agent, and an antioxidant. The dosage form of the pharmaceutical composition of the present invention is preferably a liquid preparation, and particularly preferably an injection.

The pharmaceutical composition of the present invention is used for the treatment of various malignant tumors. The malignant tumor is preferably a tumor containing cancer cells expressing HER2. Examples of the type of the malignant tumor include breast cancer, gastric cancer, colorectal cancer, salivary gland cancer, and the like.

Hereinafter, the present invention will be described in detail by way of examples, but the present invention is not limited to these examples.

### [Examples]

Example 1: Preparation of anti-HER2 antibody with modified amino acid residues in light chain FR3 Genes encoding the light chain and the heavy chain of Trastuzumab were synthesized and used as genes of the unmodified antibody. An IgG type and a Fab type of the unmodified antibody were prepared by a DNA recombination technique. In addition, IgG types and Fab types of variants were prepared using the gene of the unmodified antibody. In the Fab types of the unmodified antibody and the variants, a His tag represented by SEQ ID No. 24 was added to the C-terminus of each heavy chain for antibody purification. Details of antibody preparation are shown below.

### (1) Acquisition of plasmid for anti-HER2 antibody expression

### [Reagents]

Wizard (registered trademark) Plus SV Minipreps DNA Purification Systems (Promega Corporation)
PrimeSTAR (registered trademark)
Max DNA Polymerase (Takara Bio Inc.)
Ligation high ver.2 (Toyobo Co., Ltd.)
T4 Polynucleotide Kinase (Toyobo Co., Ltd.)
Dpn I (Toyobo Co., Ltd.)
Competent high DH5α (Toyobo Co., Ltd.)

### (1.1) Design of primers and PCR

Based on the nucleotide sequence of the synthesized gene of the unmodified antibody, eight types of primer sets were designed to obtain polynucleotides encoding light chains in which the following three amino acid residues in FR3 were substituted with arginine residues.
- Amino acid residues at positions 63, 65, and 67 of a light chain defined by the Chothia method;
- Amino acid residues at positions 63, 65, and 70 of a light chain defined by the Chothia method;
- Amino acid residues at positions 63, 65, and 72 of a light chain defined by the Chothia method;
- Amino acid residues at positions 63, 67, and 70 of a light chain defined by the Chothia method;
- Amino acid residues at positions 63, 67, and 72 of a light chain defined by the Chothia method;
- Amino acid residues at positions 65, 67, and 70 of a light chain defined by the Chothia method;
- Amino acid residues at positions 65, 67, and 72 of a light chain defined by the Chothia method; and
- Amino acid residues at positions 65, 70, and 72 of a light chain defined by the Chothia method.

The designed primer sets were synthesized, and a PCR reaction solution having the following composition was prepared. A plasmid DNA into which the gene encoding the light chain of the synthesized unmodified antibody was incorporated was used as a template.

### [PCR Reaction Solution]

| | |
|---|---|
| PrimeSTAR (registered trademark) Max DNA Polymerase | 12.5 µL |
| Forward primer (10 µM) | 1 µL |
| Reverse primer (10 µM) | 1 µL |
| Template plasmid (1 ng/µL) | 1 µL |
| Purified water | 9.5 uL |
| Total | 25 µL |

The prepared PCR reaction solution was subjected to a PCR reaction under the following reaction conditions.

### [Reaction conditions]

25 cycles of 98°C for 1 minute, 98°C for 10 seconds, 56°C for 10 seconds, and 72°C for 45 seconds, and 72°C for 3 minutes.

10 U/µL DpnI (1µL) was added to the obtained PCR product (25 µL) to fragment the PCR product. Using the DpnI-treated PCR product, a ligation reaction solution having the following composition was prepared. This reaction solution was incubated at 16°C for 1 hour to perform a ligation reaction.

| | |
|---|---|
| DpnI-treated PCR product | 2 µL |
| Ligation high ver.2 | 5 µL |
| T4 Polynucleotide kinase | 1 µL |
| Purified water | 7 µL |
| Total | 15 µL |

### (1.2) Transformation, plasmid extraction, and confirmation of sequence

The solution after the ligation reaction (2.5 µL) was added to DH5α (25 µL), and the mixture was allowed to stand on ice for 10 minutes, and then heated at 42°C for 45 seconds to perform heat shock. After allowing to stand on ice for 2 minutes again, the whole amount was applied to an ampicillin-containing LB agar medium. This agar medium was incubated at 37°C for 16 hours to obtain a transformant of E. coli. A single colony on the agar medium was taken into an ampicillin-containing LB liquid medium (2 mL) and cultured with shaking at 37°C for 16 hours. Plasmid DNA was extracted from the obtained E. coli using a QIAprep Spin Miniprep kit. The nucleotide sequence of each obtained plasmid DNA was confirmed using a pcDNA3.4 vector primer. Hereinafter, these plasmid DNAs were used as plasmid DNAs for mammalian cell expression.

### (2) Expression of unmodified antibody and variants in mammalian cells

### [Reagents]

Expi293F (trademark) cells (Thermo Fisher Scientific)
Expi293 (trademark) Expression Medium (Thermo Fisher Scientific)
ExpiFectamine (trademark) 293 Transfection Kit (Thermo Fisher Scientific)

### (2.1) Transfection

Expi293 cells were grown by shaking culture (125 rpm) under conditions of 5% CO₂ and 37°C. A number of 25 mL cell cultures (3.0 x 10⁶ cells/mL) corresponding to the number of samples were prepared. DNA solutions having the following composition were prepared using the plasmid DNA encoding each variant and the plasmid DNA encoding the unmodified antibody.

### [DNA Solution]

| | |
|---|---|
| Light chain plasmid solution | Amount (µL) corresponding to 16.7 µg |
| Heavy chain plasmid solution | Amount (µL) corresponding to 8.3 µg |
| Opti-MEM (trademark) I Reduced Serum Medium | Appropriate amount (mL) |
| Total | 1.5 mL |

Transfection reagents having the following composition were prepared and allowed to stand for 5 minutes.

| | | |
|---|---|---|
| ExpiFectamine reagent 293 Reagent | | 80 µL |
| Opti-MEM (trademark) I Reduced Serum Medium | | 1400 uL |
| Total | 1.48 mL | |

The prepared DNA solution and transfection reagent were mixed and allowed to stand for 10 minutes. The obtained mixed solution (3 mL) was added to the cell culture (25 mL), and shaking culture (125 rpm) was performed for 20 hours under conditions of 5% CO₂ and 37°C. After 20 hours, 150 µL and 1.5 mL of ExpiFectamine (trademark) Transfection Enhancers 1 and 2 were added to each culture, respectively, and shaking culture (125 rpm) was performed for 5 days under conditions of 5% CO₂ and 37°C.

### (2.2) Recovery and purification of antibody

Each cell culture was centrifuged at 3,000 rpm for 15 minutes to recover the culture supernatant. The culture supernatant contains the IgG type or Fab type of each antibody secreted from the transfected Expi293F (trademark) cells. The obtained culture supernatant was centrifuged again at 12,000 x G for 10 minutes, and the supernatant was recovered. For purification of the IgG type from the supernatant, MabSelect SuRe (trademark) (Cytiva) was used as a carrier for purification. For purification of the Fab type from the supernatant, Ni-NTA Superflow (QIAGEN) was used as a carrier for purification. Ni-NTA Superflow (100 µL) or MabSelect SuRe (100 µL) was added to each supernatant (30 mL) and reacted at 4°C overnight. The carrier was recovered, the supernatant was removed, and TBS (1 mL) or PBS (1 mL) was added to wash the carrier. To the carrier, 100 µL of TBS containing 100 mM imidazole or Arg-Antibody Elution Buffer (pH 4.0) (Nacalai Tesque, Inc.) was added to elute the antibody from each carrier. This elution operation was performed three times in total to obtain an antibody solution.

The amino acid sequences of the light chains of the IgG type and Fab type variants and unmodified antibody were as follows.
- 63,65,67 variant: SEQ ID No. 2
- 63,65,72 variant: SEQ ID No. 4
- 63,67,72 variant: SEQ ID No. 6
- 65,67,72 variant: SEQ ID No. 8
- 63,65,70 variant: SEQ ID No. 20
- 63,67,70 variant: SEQ ID No. 21
- 65,67,70 variant: SEQ ID No. 22
- 65,70,72 variant: SEQ ID No. 23
- Unmodified antibody: SEQ ID No. 10

The amino acid sequences of the heavy chains of the IgG type variants and unmodified antibody were all the amino acid sequence of SEQ ID No. 12. The amino acid sequences of the heavy chains of the Fab type variants and unmodified antibody were all the amino acid sequence of SEQ ID No. 25. The amino acid sequence of SEQ ID No. 25 is shown below.

EVQLVESGGGLVQPGGSLRLSCAASGFNIKDTYIHWVRQAPGKGLEWVARIYPTNG
YTRYADSVKGRFTISADTSKNTAYLQMNSLRAEDTAVYYCSRWGGDGFYAMDYWGQGTLV
TVSSASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKKVEPKSCDKTGSCGGHHHHHH (SE
Q ID No. 25)

### Example 2: Confirmation of affinity of variants for HER2

### (1) Analysis of affinity between antibody and antigen

The affinity of the unmodified antibody and each variant prepared in Example 1 for HER2 was measured using Biacore (registered trademark) 8k (Cytiva). The Fab type of each antibody was used for the measurement. Recombinant Human ErbB2/Her2 Fc Chimera Protein, CF (R&D Systems; 1129-ER) was used as an antigen. The above antigen was immobilized on a Sensor Chip CM4 for Biacore (registered trademark) (Cytiva) by a capture method using a Human capture kit (Cytiva). The antibody solution was diluted to adjust the antibody concentration to 5 nM, 2.5 nM, 1.25 nM, 0.625 nM, and 0.3125 nM. The antibody solution of each concentration was sent to Biacore (registered trademark) 8k (Cytiva). The measurement data was analyzed using Biacore (registered trademark) Evaluation software to obtain data on the affinity of each antibody.

### (2) Results

As a result of the analysis, there was no variant whose affinity for the antigen was lower than that of the unmodified antibody. Therefore, it was shown that all of the variants prepared in Example 1 can be used as anti-HER2 antibodies in the same manner as the unmodified antibody.

Example 3: Analysis of non-specific adsorption of variants to HER2-negative cells Non-specific adsorption of each variant prepared in Example 1 was analyzed using mouse-derived PBMC as HER2-negative cells.

### (1) Flow cytometry analysis

BALB/c mouse PBMC (IQ BIOSCIENCES) was added to a solution (100 µg/mL) of each variant of IgG type and brought into contact for 1 hour. Thereafter, the cells were washed with 0.5% BSA/PBS. After washing, FITC-labeled anti-human IgG Fc (Bethyl Laboratories) and 7-AAD (BioLegend) were added to the cells and reacted at room temperature for 20 minutes. The FITC-labeled anti-human IgG Fc was a labeled secondary antibody capable of binding to the IgG type variant. 7-AAD was a nuclear staining dye enabling discrimination between live cells and dead cells. For background measurement, PBMC not in contact with the variant was reacted with FITC-labeled anti-human IgG Fc and 7-AAD. After the reaction, the cells were measured with a flow cytometer iQue (registered trademark) 3 (Sartorius) to obtain fluorescence intensity derived from FITC for live cells. iQue Forecyt (registered trademark) (Sartorius) was used for data analysis.

### (2) Results

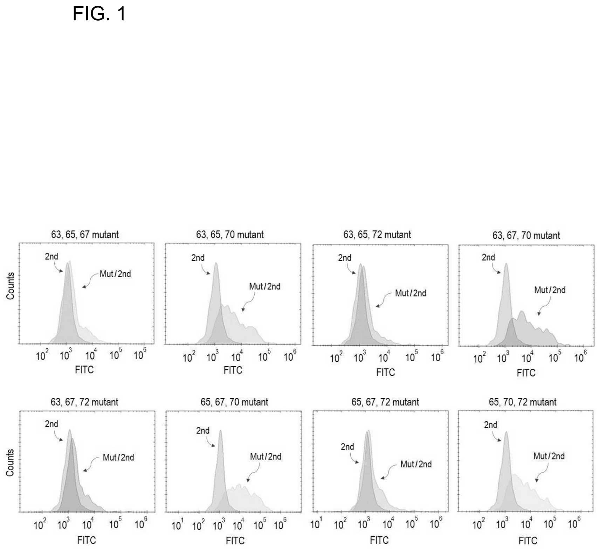

For each variant, a histogram was created based on the obtained data, with the horizontal axis representing fluorescence intensity (FITC) and the vertical axis representing the number of cells (Counts). The results are shown in Fig. 1. In the figure, "2nd" indicates a histogram of cells contacted only with the labeled secondary antibody. "Mut/2nd" indicates a histogram of cells contacted with the variant and the labeled secondary antibody. In Fig. 1, the histogram of 2nd and the histogram of Mut/2nd were superimposed and displayed. As can be seen from Fig. 1, when the 63,65,67 variant, the 63,65,72 variant, the 63,67,72 variant, and the 65,67,72 variant were used, the histogram of Mut/2nd almost overlapped with the histogram of 2nd. On the other hand, when the 63,65,70 variant, the 63,67,70 variant, the 65,67,70 variant, and the 65,70,72 variant were used, the histogram of Mut/2nd was shifted to the high fluorescence intensity side compared to the histogram of 2nd.

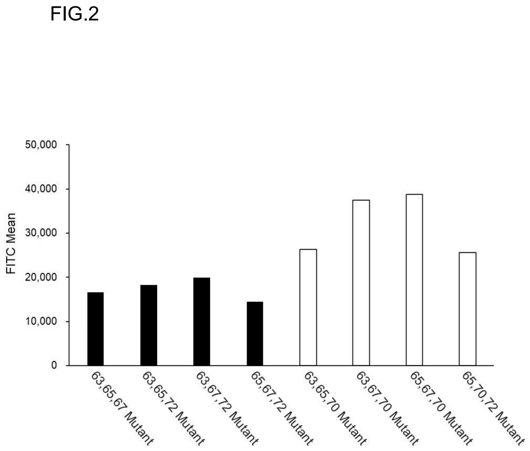

In order to examine the increase in fluorescence intensity due to non-specific adsorption to PBMC, the average value of fluorescence intensity was obtained based on the histogram of Mut/2nd for each variant shown in Fig. 1. Fig. 2 shows the average value of fluorescence intensity of each variant. As shown in Fig. 2, the 63,65,67 variant, the 63,65,72 variant, the 63,67,72 variant, and the 65,67,72 variant had lower average values of fluorescence intensity than the 63,65,70 variant, the 63,67,70 variant, the 65,67,70 variant, and the 65,70,72 variant. From these results, it was suggested that non-specific adsorption to PBMC is suppressed in the 63,65,67 variant, the 63,65,72 variant, the 63,67,72 variant, and the 65,67,72 variant compared to the 63,65,70 variant, the 63,67,70 variant, the 65,67,70 variant, and the 65,70,72 variant.

### Example 4: Analysis of non-specific adsorption of variants to heparin column

Non-specific adsorption of each variant prepared in Example 1 was analyzed using a heparin column.

### (1) Chromatography analysis

The IgG type variant (100 µg) was applied to HiTrap (trademark) Heparin HP 1 mL (Cytiva) equilibrated with eluent A (20 mM Tris-HCl, pH 7.5). After washing the column with buffer A, eluent B (20 mM Tris-HCl, 1 M NaCl, pH 7.5) was increased from 0% to 100%. Thereby, the antibody adsorbed to the column was gradient eluted. The analysis conditions were as follows.
Analyzer: AKTA pure (trademark) 150M (Cytiva)
Detector: Ultraviolet absorptiometer Analysis
Condition: Measure wavelength of 280 nm
Column: HiTrap Heparin HP 1 mL
Temperature: Room temperature
Flow rate: 1.0 mL/min
Sample: Purified antibody 100 µg
Eluent A: 20 mM Tris-HCl, pH 7.5
Eluent B: 20 mM Tris-HCl, 1 M NaCl, pH 7.5

### Gradient conditions

| Time after injection (min) | Eluent A (vol %) | Eluent B (vol %) | Remarks |
|---|---|---|---|
| 0-5 | 100 | 0 | |
| 5-15 | 100-0 | 0-100 | Linear gradient |
| 15-20 | 0 | 100 | |

### (2) Results

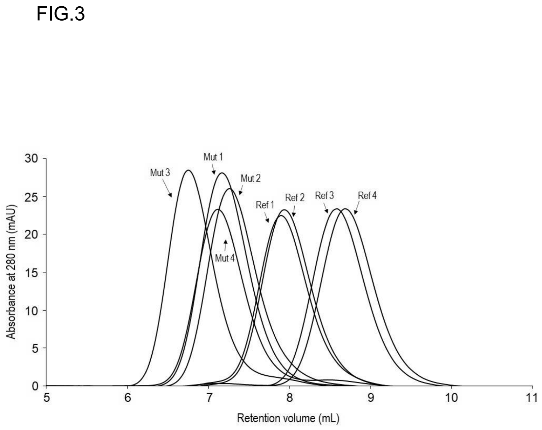

The retention volume (mL) of each variant is shown in Fig. 3. In the figure, "Mut 1" indicates the 63,65,67 variant, "Mut 2" indicates the 63,65,72 variant, "Mut 3" indicates the 63,67,72 variant, "Mut 4" indicates the 65,67,72 variant, "Ref 1" indicates the 63,65,70 variant, "Ref2" indicates the 63,67,70 variant, "Ref 3" indicates the 65,67,70 variant, and "Ref 4" indicates the 65,70,72 variant. As can be seen from Fig. 3, the 63,65,67 variant, the 63,65,72 variant, the 63,67,72 variant, and the 65,67,72 variant had lower retention volumes compared to the 63,65,70 variant, the 63,67,70 variant, the 65,67,70 variant, and the 65,70,72 variant. From these results, it was suggested that non-specific adsorption to the heparin column is suppressed in the 63,65,67 variant, the 63,65,72 variant, the 63,67,72 variant, and the 65,67,72 variant compared to the 63,65,70 variant, the 63,67,70 variant, the 65,67,70 variant, and the 65,70,72 variant.

## Claims

1. An isolated anti-HER2 antibody, in which three amino acid residues selected from the group consisting of positions 63, 65, 67, and 72 of a light chain defined by the Chothia method are arginine residues.

2. The anti-HER2 antibody according to claim 1, wherein said antibody has been obtained by changing three amino acid residues selected from the group consisting of positions 63, 65, 67, and 72 of a light chain defined by the Chothia method to arginine in an anti-HER2 antibody in which, in its original sequence, three amino acid residues selected from the group consisting of positions 63, 65, 67, and 72 of a light chain defined by the Chothia method are not arginine;
wherein the non-specific adsorption to a HER2-negative cell of the anti-HER2 antibody is lower than that of a reference antibody, and
the reference antibody comprises a light chain represented by an amino acid sequence of SEQ ID No. 20, 21, 22, or 23 and a heavy chain represented by an amino acid sequence of SEQ ID No. 12 or 13, and the amino acid sequences of the light chain and the heavy chain are defined by the Chothia method.

3. The anti-HER2 antibody according to claim 2, wherein said three amino acid residues are changed from neutral amino acid residues to arginine residues.

4. The anti-HER2 antibody according to claim 3, wherein the neutral amino acid residues are threonine residues and/or serine residues.

5. The anti-HER2 antibody according to any of claim 1, wherein
the light chain comprises LCDR1 represented by an amino acid sequence of SEQ ID No. 14, LCDR2 represented by an amino acid sequence of SEQ ID No. 15, and LCDR3 represented by an amino acid sequence of SEQ ID No. 16, and
the heavy chain comprises HCDR1 represented by an amino acid sequence of SEQ ID No. 17, HCDR2 represented by an amino acid sequence of SEQ ID No. 18, and HCDR3 represented by an amino acid sequence of SEQ ID No. 19.

6. The anti-HER2 antibody according to any of claims 1 to 5, comprising a light chain variable region represented by an amino acid sequence of SEQ ID No. 1, 3, 5, or 7 and a heavy chain variable region represented by an amino acid sequence of SEQ ID No. 11.

7. The anti-HER2 antibody according to any of claims 1 to 6, wherein an amino acid sequence of the light chain is an amino acid sequence of SEQ ID No. 2, 4, 6, or 8, and an amino acid sequence of the heavy chain is an amino acid sequence of SEQ ID No. 12 or 13.

8. A method for producing an anti-HER2 antibody, comprising
generating an anti-HER2 antibody comprising an amino acid sequence of the anti-HER2 antibody using a polynucleotide encoding the amino acid sequence of the anti-HER2 antibody, and recovering the anti-HER2 antibody,
wherein in the polynucleotide, codons encoding three amino acid residues selected from the group consisting of positions 63, 65, 67, and 72 of a light chain defined by the Chothia method are codons encoding arginine residues.

9. The method according to claim 8, wherein the anti-HER2 antibody comprises a light chain variable region represented by an amino acid sequence of SEQ ID No. 1, 3, 5, or 7 and a heavy chain variable region represented by an amino acid sequence of SEQ ID No. 11.

10. The method according to claim 8 or 9, wherein an amino acid sequence of the light chain is an amino acid sequence of SEQ ID No. 2, 4, 6, or 8, and an amino acid sequence of the heavy chain is an amino acid sequence of SEQ ID No. 12 or 13.

11. A method for suppressing non-specific adsorption of an anti-HER2 antibody, comprising
suppressing non-specific adsorption to a HER2-negative cell of the antibody compared to a reference antibody by changing three amino acid residues in framework region 3 selected from the group consisting of positions 63, 65, 67, and 72 of a light chain defined by the Chothia method to arginine residues in the anti-HER2 antibody,
wherein the non-specific adsorption is lower than that of a reference antibody and
the reference antibody comprises a light chain represented by an amino acid sequence of SEQ ID No. 20, 21, 22, or 23 and a heavy chain represented by an amino acid sequence of SEQ ID No. 12 or 13, and the amino acid sequences of the light chain and the heavy chain are defined by the Chothia method.

12. The method according to claim 11, wherein the method comprises suppressing non-specific absorption of an anti-HER2 antibody having an original amino acid sequence and the original amino acid sequence of a variable region of the light chain of said anti-HER antibody is an amino acid sequence of SEQ ID No. 9, and an amino acid sequence of a variable region of the heavy chain is an amino acid sequence of SEQ ID No. 11.

13. The method according to any of claim 11 or 12, wherein the method comprises suppressing non-specific absorption of an anti-HER2 antibody having an original amino acid sequence and wherein the original amino acid sequence of the light chain is an amino acid sequence of SEQ ID No. 10, and an amino acid sequence of the heavy chain is an amino acid sequence of SEQ ID No. 12 or 13.

14. The method according to any one of claims 10 to 13, wherein the anti-HER2 antibody obtained by said method comprises a light chain variable region represented by an amino acid sequence of SEQ ID No. 1, 3, 5, or 7 and a heavy chain variable region represented by an amino acid sequence of SEQ ID No. 11.

15. The method according to any one of claims 10 to 14, wherein an amino acid sequence of the light chain of the antibody obtained by said method is an amino acid sequence of SEQ ID No. 2, 4, 6, or 8, and an amino acid sequence of the heavy chain is an amino acid sequence of SEQ ID No. 12 or 13.
